# EUROPEAN PATENT SPECIFICATION

(11) **EP 2 111 865 B1**
(45) Date of publication and mention of the grant of the patent: **02.10.2019**
(21) Application number: 07739883.2
(22) Date of filing: 27.03.2007
(51) Int. Cl.: A61K 31/7004, A61P 3/04, A61P 3/06, A61P 3/10, A61K 31/121, C07H 3/02

(54) **UTILIZATION OF THE FUNCTION OF RARE SUGAR AS PROMOTER FOR THE MIGRATION OF GLUCOKINASE FROM NUCLEUS TO CYTOPLASM**
AUSNUTZUNG DER FUNKTION VON SELTENEN ZUCKERN ALS MITTEL ZUR FÖRDERUNG DER MIGRATION VON GLUCOKINASE AUS DEM NUCLEUS IN DAS ZYTOPLASMA
UTILISATION DE LA FONCTION DE SUCRE RARE EN TANT QUE PROMOTEUR DE LA MIGRATION DE GLUCOKINASE DEPUIS LE NOYAU JUSQU'AU CYTOPLASME

(30) Priority: 17.11.2006 JP 2006310982
(43) Date of publication of application: 28.10.2009
(73) Proprietor: National University Corporation Kagawa University, Takamatsu-shi, Kagawa 760-8521 (JP); Matsutani Chemical Industry Co., Ltd., Itami-shi Hyogo 664-8508 (JP)
(72) Inventor: TOKUDA, Masaaki, Kita-gun Kagawa 761-0793 (JP); IZUMORI, Ken, Kita-gun Kagawa 761-0793 (JP); TOYODA, Yukiyasu, Nagoya-shi Aichi 466-0834 (JP); MIWA, Ichitomo, Nishikamo-gun Aichi 470-0202 (JP)
(74) Representative: TBK
(86) International application number: PCT/JP2007/056445
(87) International publication number: WO 2008/059625

(56) References cited:
- WO-A1-02/058710
- WO-A1-03/097820
- WO-A1-2006/101118
- WO-A1-2007/010975
- WO-A1-2007/010976
- JP-A- 02 062 887
- JP-A- 06 065 080
- JP-A- 2005 213 227
- JP-A- 2007 051 137
- US-A- 5 356 879
- US-A- 5 447 917
- Donner: "D-tagatose, a novel hexose: acute effects on carbohydrate tolerance in subjects with and without type 2 diabetes" vol. 1, no. 5, 25 December 2001 (2001-12-25), pages 1/4-4/4, XP002587708 Retrieved from the Internet: URL:http://www3.interscience.wiley.com/cgi -bin/fulltext/120194037/HTMLSTART [retrieved on 2010-06-17]
- LEVIN GILBERT V: "Tagatose, the new GRAS sweetener and health product." JOURNAL OF MEDICINAL FOOD SPRING 2002 LNKD- PUBMED:12511110, vol. 5, no. 1, April 2002 (2002-04), pages 23-36, XP002587709 ISSN: 1096-620X
- MATSUO T ET AL: "Less Body Fat Accumulation with D-Psicose Diet versus D-Fructose Diet" JOURNAL OF CLINICAL BIOCHEMISTRY AND NUTRITION, TOKYO, JP, vol. 30, 1 January 2001 (2001-01-01), pages 55-65, XP003003027 ISSN: 0912-0009
- AGIUS L: "Control of glucokinase translocation in rat hepatocytes by sorbitol and the cytosolic redox state." THE BIOCHEMICAL JOURNAL 15 FEB 1994 LNKD- PUBMED:8129726, vol. 298 ( Pt 1), 15 February 1994 (1994-02-15), pages 237-243, XP002587710 ISSN: 0264-6021
- MOORE M.C.: 'Drug evaluation: tagatose in the treatment of type 2 diabetes and obesity' CURRENT OPINION IN INVESTIGATIONAL DRUGS vol. 7, no. 10, October 2006, pages 924 - 935, XP003022570
- MATSUO T. ET AL.: 'Effects of dietary D-psicose on diurnal variation in plasma glucose and insulin concentrations of rats' BIOSCIENCE, BIOTECHNOLOGY, AND BIOCHEMISTRY vol. 70, no. 9, September 2006, pages 2081 - 2085, XP003022571
- TOKUDA M.: 'Iyaku to shite no Kishoto no Oyo (Application of rare sugars for medical purposes)' THE SOCIETY FOR BIOTECHNOLOGY TAIKAI KOEN YOSHISHU 2005, page 25, XP003022572

## Description

### Technical Field

The present invention relates to a composition containing as the active ingredients rare sugars D-psicose **or** D-psicose and D-tagatose for use in preventing the onset of disordered conditions in association with glucokinase activity and therapeutically treating the disordered conditions.

### Background Art

Glucokinase (EC 2.7.1.1) is one of four hexokinase types discovered in mammalians. Hexokinases catalyze the first step of the D-glucose metabolism, namely the conversion of glucose to D-glucose 6-phosphate. Glucokinase has a limited cellular distribution and is mainly observed in for example pancreatic β cells, hepatocytes, cerebral hypothalamus and intestinal tube.

Liver is an important organ for the maintenance of the homeostasis of blood glucose. Glucokinase as one of the glycolytic rate-limiting enzymes in the sugar metabolism in liver binds to the glucokinase regulatory protein in the cell nucleus during fasting and therefore exists as the inactive type therein. Due to the increase of extracellular D-glucose concentration and the existence of D-fructose at a lower concentration, glucokinase is dissociated from the glucokinase regulatory protein and is then transferred from the nucleus into the cytoplasm, where glucokinase promotes the D-glucose metabolism. The transfer of glucokinase between nucleus and cytoplasm is regulated by hormones. Insulin transfers glucokinase from nucleus to cytoplasm, while glucagon transfers glucokinase from cytoplasm to nucleus.

So as to block hypoglycemia in normal subjects or in individuals with diabetic mellitus, liver generates D-glucose. Such D-glucose generation is induced by the release of D-glucose from glycogen under storage or by gluconeogenesis (de novo intracellular synthesis of D-glucose from a gluconeogenetic precursor, which is a process mediated with the enzyme D-glucose-6-phosphatase). However, the deterioration of sugar utilization in liver and the elevation of gluconeogenesis are observed in type 2 diabetic mellitus. In case that D-glucose generation in liver cannot appropriately be regulated and/or is increased, generated D-glucose during fasting sometimes amounts to 2-fold or more. Additionally, some of patients with impaired glucose tolerance (a provisional group of diabetes mellitus) or some of patients with type 2 diabetes mellitus may sometimes be at high blood glucose levels after meals even though their blood glucose levels are normal during fasting. Insufficient insulin secretion and insulin resistance may cause abnormalities in the sugar utilization or/and gluconeogenesis in liver, as well as the deterioration of sugar utilization in muscles and fat tissues, which leads to the increase of blood glucose levels after meals.

Additionally, glucokinase also exists in cerebral hypothalamus. It is understood that glucokinase functions as a glucose sensor therein. The existence of the glucokinase regulatory protein even in cerebral hypothalamus and the existence of glucokinase in the binding type and the free type may cause the enhancement of glucokinase activity due to the transfer of glucokinase from the nucleus to the cytoplasm with rare sugars in hypothalamus or the increase of glucokinase activity owing to the increase of the free-type glucokinase from the binding-type glucokinase. Such modification may be transmitted through the nerve system to peripheral tissues, where the elevation of glucose utilization in the peripheral tissues and the suppression of dietary intake occur. In such fashion, glucokinase is efficacious in the prevention and therapeutic treatment of impaired glucose tolerance, diabetes mellitus, obesity and the metabolic syndrome.

In pancreatic β cells, glucose exists in the cytoplasm and also partially exists in the insulin-secreting granules, not in the nucleus . Therefore, it is expected that glucokinase may also be useful for preventing and therapeutically treating impaired glucose tolerance and diabetes mellitus, through the increase of insulin secretion owing to the enhancement of glucokinase activity with rare sugars even in pancreatic β cells.

A glucokinase-activating agent has been reported in recent years, which works for increasing glucokinase activity and therefore exerts a hypoglycemic action.

The glucokinase-activating agent increases the glucose metabolic rate in the β cells and hepatocytes, which is associated with the increase of insulin secretion. Such pharmaceutical agent will be useful for therapeutic treatment of type 2 diabetes mellitus.

As such glucokinase-activating agent, there are proposed the gene encoding glucokinase, D-glucose, cAMP, retinoic acid and the like (patent reference 1); substituted phenylacetamide (patent reference 2); heteroarylcarbamoylbenzene derivatives (patent reference 3) ; aminobenzamide derivatives (patent reference 4); and pyridine-2-carboxamide derivatives (patent reference 5).

Furthermore, a method for producing rare sugars at a mass scale has been developed, so attention has now been focused on the functions.
Patent reference 1: JP-A-2004-018403
Patent reference 2: Domestic Re-publication of PCT Application 2003-532718
Patent reference 3: Domestic Re-publication of PCT Application 2004-076420
Patent reference 4: Domestic Re-publication of PCT Application 2003-080585
Patent reference 5: Domestic Re-publication of PCT Application 2004-080001

MOORE M.C., "Drug evaluation: tagatose in the treatment of type 2 diabetes and obesity", CURRENT OPINION IN INVESTIGATIONAL DRUGS, (200610), vol. 7, no. 10, pages 924 - 935, XP003022570 describes the investigation of tagatose as an orally active lactose derivative for the potential treatment of obesity and type 2 diabetes.

WO 2006/101118 A1 is directed at the use of D-psicose for suppressing abnormal circadian increase in plasma glucose level.

MATSUO T ET AL, "Less Body Fat Accumulation with D-Psicose Diet versus D-Fructose Diet", JOURNAL OF CLINICAL BIOCHEMISTRY AND NUTRITION, TOKYO, JP, (20010101), vol. 30, ISSN 0912-0009, pages 55 - 65, XP003003027, is a study about the effects on body fat accumulation of D-psicose compared with cellulose or D-fructose in rats.

MATSUO T. ET AL., "Effects of dietary D-psicose on diurnal variation in plasma glucose and insulin concentrations of rats", BIOSCIENCE, BIOTECHNOLOGY, AND BIOCHEMISTRY, (200609), vol. 70, no. 9, pages 2081 - 2085, XP003022571, is a study about the effects of supplemental D-psicose in the diet on diurnal variation in plasma glucose and insulin concentrations in rats.

### Disclosure of the Invention

### Problems that the Invention is to Solve

For the purpose of developing a therapeutic agent of diabetes mellitus via the promotion of the glucokinase transfer from the nucleus to the cytoplasm in liver, the inventors made examinations about the signal transduction system for the glucokinase transfer between the nucleus and the cytoplasm in liver with insulin and glucagon; for the purpose of screening for a sugar functioning in place of D-fructose, the inventors made examinations about the influence of a rare sugar ketose on the glucokinase transfer between the nucleus and the cytoplasm, at a second step.

Specifically, the inventors made screenings for a glucokinase-activating substance among rare sugars. It is an object of the invention to provide a composition containing as the active ingredient the glucokinase-activating substance for treating (preventing and therapeutically treating) disordered conditions in association with glucokinase activity (= in association with the promotion of glucokinase transfer between nucleus and cytoplasm) . It is an additional object of the invention to provide a technique for using rare sugars D-psicose and/or D-tagatose capable of treating (preventing and therapeutically treating) disordered conditions in association with glucokinase activity, via simple ingestion or oral administration thereof in forms of food additives, food materials, foods and drinks, health foods and drinks, pharmaceutical products and feeds.

### Means for Solving the Problems

The invention relates to a composition for uses as defined in the claims.

### Advantages of the Invention

In other words, a promoting agent of glucokinase transfer from nucleus to cytoplasm, namely D-psicose or D-psicose and D-tagatose could be found among rare sugars in accordance with the invention. In accordance with the invention, specifically, there can be provided
a composition which contains D-psicose or D-psicose and D-tagatose as the active ingredient for use in preventing the onset of disordered conditions in assocication
with glucokinase activity (= in association with glucokinase transfer from nucleus to cytoplasm) and therapeutically treating the disordered conditions as defined in claim 1. More specifically, in accordance with the invention, there can be provided a technique for using rare sugars D-psicose or D-psicose and D-tagatose capable of treating (preventing and therapeutically treating) disordered conditions in associations with glucokinase activity, via simple ingestion or oral administration thereof in forms of food additives, food materials, foods and drinks, health foods and drinks, pharmaceutical products and feeds.

### Brief Description of the Drawings

[Fig. 1] A scheme describing glucokinase.
[Fig. 2] Graphs describing the influence of the D-fructose concentration on the glucose phosphorylation rate in cultured hepatocytes.
[Fig. 3] A scheme describing the regulation mechanism of glucokinase activity with the glucokinase regulatory protein in hepatocytes.
[Fig. 4] Photographs and graphs showing the assay results of glucokinase distribution in hepatocytes by a fluorescence antibody method with an anti-glucokinase antibody.
[Fig. 5] A scheme describing the mechanism of the sugar metabolism with glucokinase transfer between the nucleus and the cytoplasm in liver and the disorders in type 2 diabetes mellitus.
[Fig. 6] Photographs and bar graphs showing the change of the glucokinase distribution in hepatocytes 30 minutes after the oral administration of D-glucose or D-fructose.
[Fig. 7] Graphs showing the ratio of glucokinase existing in cytoplasm on the transfer promotion with D-fructose at a low concentration.
[Fig. 8] Graphs showing the change of blood glucose levels in portal vein and peripheral blood in a model rat of type 2 diabetes mellitus and a normal rat.
[Fig. 9] A scheme describing that a small amount of D-fructose administered to a rat or a patient with type 2 diabetes mellitus can suppress the increase of blood glucose levels therein.
[Fig. 10] Graphs showing the effects of D-ketose on the glucokinase transfer from nucleus to cytoplasm.
[Fig. 11] Graphs showing the effects of L-ketose on the glucokinase transfer from nucleus to cytoplasm.
[Fig. 12] Graphs describing the effects of D-ketose on lactic acid generation.
[Fig. 13] Graphs showing the effects of D-psicose on the glucokinase transfer from nucleus to cytoplasm.
[Fig. 14] A scheme showing protocols for animal experiments so as to verify the transfer of glucokinase from nucleus to cytoplasm.
[Fig. 15] Photographs showing the glucokinase distribution in hepatocytes in the series of Fig.14, which was assayed by a fluorescence antibody method with an anti-glucokinase antibody, where the results obtained are shown for comparison.
[Fig. 16] A scheme showing protocols for animal experiments so as to confirm the transfer of glucokinase from nucleus to cytoplasm in series with combinations of D-glucose, D-psicose and D-fructose.
[Fig. 17] Photographs showing the glucokinase distribution in hepatocytes in the series of Fig.16, which was assayed by a fluorescence antibody method with an anti-glucokinase antibody, where the results obtained are shown for comparison.
[Fig. 18] A scheme showing protocols for experiments so as to examine the glucokinase transfer from nucleus to cytoplasm in Goto-Kakizaki rats as a model animal of type 2 diabetes mellitus and examine the change of the blood glucose levels and the insulin concentration in the tail vein and the portal vein.
[Fig. 19] Photographs showing the glucokinase distribution in hepatocytes in the series of Fig.18, which was assayed by a fluorescence antibody method with an anti-glucokinase antibody, where the results obtained are shown for comparison with the assayed results of the glucokinase distribution in nucleus and cytoplasm.
[Fig. 20] Graphs showing the assay results of the glucokinase levels in cytoplasm as determined by the analysis of the fluorescence images in Fig.19, which are shown for comparison.
[Fig. 21] Graphs showing the assay results of blood glucose levels in the tail vein and the portal vein in the series of Fig.18, which are shown for comparison.
[Fig. 22] Graphs showing the assay results of insulin concentrations in the tail vein and the portal vein in the series of Fig.18, which are shown for comparison.

### Best Mode for Carrying out the Invention

In accordance with the disclosure, a compound activating glucokinase by binding to the allosteric site of glucokinase is defined as "a glucokinase-activating agent"; a compound promoting the transfer of liver glucokinase between nucleus and cytoplasm is defined as "a promoting agent of glucokinase transfer from nucleus to cytoplasm".

Additionally, the "promoting agent of glucokinase transfer from nucleus to cytoplasm" never means that the transfer is limited to glucokinase transfer from nucleus to cytoplasm but includes compounds inducing the increase of the active-type glucokinase from inactive-type glucokinase, namely agents of increasing the active-type glucokinase.

Possibly, the increase of the active-type glucokinase from the binding-type glucokinase may cause some change in both hypothalamus and pancreatic β cells, which is supported in Examples of the invention.

Cytoplasmic glucokinase or glucokinase transferred from nucleus to cytoplasm interacts with cytoplasmic proteins and intracellular small organs. For example, glucokinase may possibly bind to fructose 2,6-bisphosphate synthase and decomposition enzymes responsible for the regulation of glycolytic enzymes, i.e. glucokinase may possibly be involved in glycolytic regulation. Additionally, glucokinase may possibly bind to other proteins to increase the glucokinase activity. The possibilities may suggest a possibility that glucokinase may bind to mitochondria. Among others, the publication of JP-A-2001-333778 discloses that the glucokinase activity increases when glucokinase binds to propionyl CoA carboxylase existing in mitochondria. However, no experiments are reported in the publication to confirm that D-psicose and other rare sugars would bring about such effect. In accordance with the invention, this possibility is supported in Examples.

With attention focused on hypothalamus and pancreatic β cells as described in the Background Art, the above possibility is now described.

It is defined that the "promoting agent of glucokinase transfer from nucleus to cytoplasm" never means that the transfer is limited to glucokinase transfer from nucleus to cytoplasm but includes compounds inducing the increase of the active-type glucokinase from the inactive-type glucokinase, namely agents of increasing the active-type glucokinase, so as to encompass the significance such that the interaction of glucokinase with intracellular small organs or intracellular proteins to cause the modification of the glucokinase activity and the modification of the functions of proteins interactive with intracellular small organs and glucokinase can regulate the intracellular metabolisms and the cell functions.

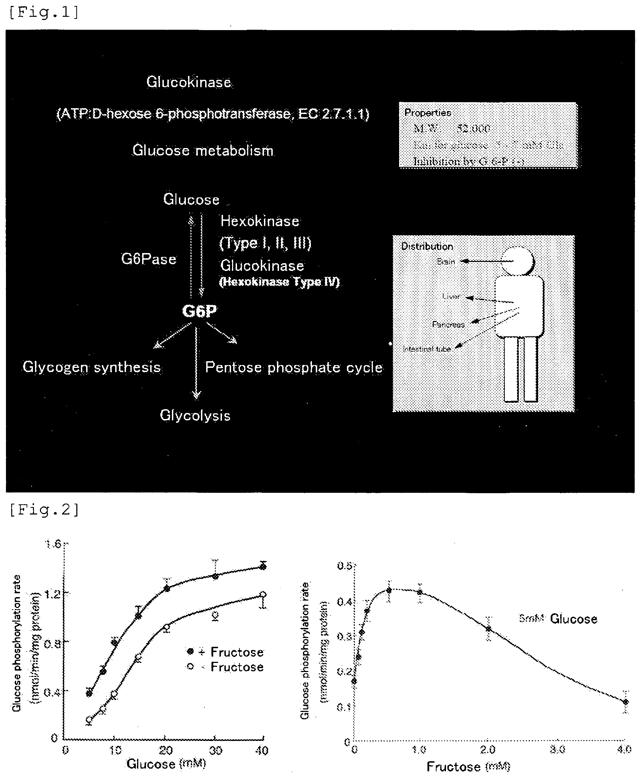

Fig. 1 is a scheme describing glucokinase.

As shown in Fig. 1, hexokinase is an enzyme converting D-glucose to D-glucose 6-phosphate. Hexokinase includes four types of isozymes (type I, type II, type III and type IV), while the hexokinase type IV is glucokinase. Differing from the other isozymes (hexokinase types I through III), glucokinase has a low affinity with glucose (Km; 5 to 10 mM), without any feed back inhibition with D-glucose 6-phosphate, unlike the other isozymes (hexokinase types I, II, and III). The enzyme distributes in pancreatic Langerhans Island, liver, brain and intestinal tube. This indicates that glucokinase has an important role in the homeostasis of blood glucose in biological organisms.

It has been demonstrated that glucokinase in the β cell of pancreas Langerhans Island is a glucose sensor regulating insulin secretion on D-glucose stimulation.

The glucokinase functions to suppress the increase of blood glucose level after meal in liver, by promoting sugar utilization therein.

Since glucokinase exists in the nerve nucleus of hypothalamus as the central system of the autonomic nerve or in the ependyma cells of ventricule wall as well, it is understood that the enzyme functions as a glucose sensor detecting the D-glucose concentration in cerebral parenchyma and cerebrospinal fluid.

Fig. 2 shows graphs describing the influence of D-fructose concentration on the glucose phosphorylation rate in cultured hepatocytes.

It is known that fructose at a low concentration promotes glucose phosphorylation in liver. The results are shown in Fig. 2.

A primary culture of rat hepatocytes was incubated at various glucose concentrations in the presence and absence of 0.5 mM D-fructose. The existence of fructose enhanced glucose phosphorylation in the hepatocytes (the left view of Fig.2). Subsequently, the primary culture was incubated at various D-fructose concentrations in the presence of 25 mM D-glucose. Up to 0.5 mM D-fructose concentration, glucose phosphorylation was enhanced as the D-fructose concentration was raised. Above the concentration, inversely, glucose phosphorylation was reduced (the right view of Fig.2). The results agreed with the results reported so far.

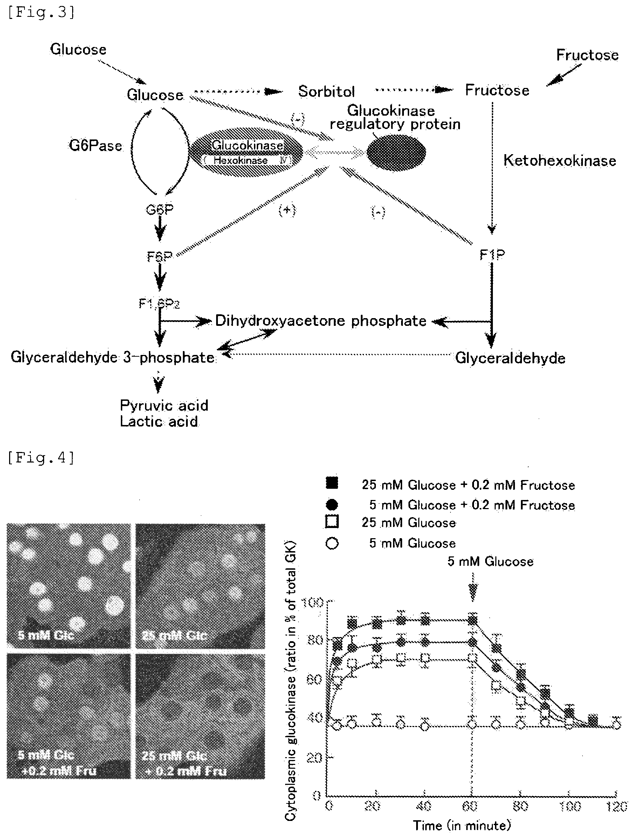

Fig. 3 is a scheme describing the regulation mechanism of glucokinase activity with the glucokinase regulatory protein in hepatocytes.

The finding that the glucose metabolism in liver increased via the presence of a low concentration of D-fructose (up to 0.5 mM) revealed the presence of a glucokinase regulatory protein regulating the activity of glucokinase through the binding to glucokinase in hepatocytes.

Fig. 3 collectively shows the regulation mechanism of the glucokinase activity with the glucokinase regulatory protein. The glucokinase regulatory protein binds to glucokinase to inhibit the activity thereof. The affinity between glucokinase and the glucokinase regulatory protein is enhanced with D-fructose 6-phosphate but is reduced with D-fructose 1-phosphate or with a high concentration of glucose. This indicates that glucokinase when bound to the glucokinase regulatory protein is in the inactive type and glucokinase when dissociated from the glucokinase regulatory protein is in the active type.

When D-glucose concentration is increased outside hepatocytes after meal, the intracellular glucose concentration is increased, to cause the dissociation of glucokinase from the glucokinase regulatory protein, so that glucokinase turns from the inactive type to the active type, inducing the enhancement of glucose phosphorylation and the increase of D-glucose utilization. When a low concentration of D-fructose exists extracellularly, D-fructose is converted with ketohexokinase to D-fructose 1-phosphate. The generated D-fructose 1-phophate dissociates glucokinase from the glucokinase regulatory protein, so that glucokinase turns from the inactive type to the active type, as in the case of D-glucose, so that glucose phosphorylation is enhanced, while D-glucose utilization is also increased.

Fig. 4 shows photographs and graphs showing the assay results of the glucokinase distribution in hepatocytes as assayed by a fluorescence antibody method with an anti-glucokinase antibody.

Agius et al. in England found that glucokinase was in association with a cell backbone protein in hepatocytes. They found that glucokinase was dissociated from the cell backbone protein due to the increase of extracellular glucose concentration and the existence of D-fructose at a low concentration.

The inventors assayed the glucokinase distribution in hepatocytes by a fluorescence antibody method with an anti-glucokinase antibody, and found that glucokinase mainly existed in the cell nucleus. Additionally, the inventors found that D-glucose orally administered to a rat transferred glucokinase from nucleus to cytoplasm.

Fig. 4 shows the results of the glucokinase distributions assayed by a fluorescence antibody method, by incubating a primary culture of hepatocytes in the presence of 5 mM D-glucose, 25 mM D-glucose, 5 mM D-glucose + 0.5 mM D-fructose, or 25 mM D-glucose + 0.5 mM D-fructose at 37°C for 30 minutes and then fixing the hepatocytes with 4 % para-formaldehyde. Green fluorescence shows glucokinase distribution. In the presence of 5 mM D-glucose, glucokinase mainly existed in the cell nucleus. The nuclear glucokinase was reduced via the incubation with 25 mM D-glucose, while the cytoplasmic glucokinase was increased. In the presence of 0.5 mM D-fructose compared with the absence of D-fructose, the glucokinase transfer from the nucleus to the cytoplasm increased.

The glucokinase regulatory protein was also examined about the distribution thereof in hepatocytes with the fluorescence antibody method using an anti-glucokinase regulatory antibody. The inventors found that the glucokinase regulatory protein also existed in the nucleus.

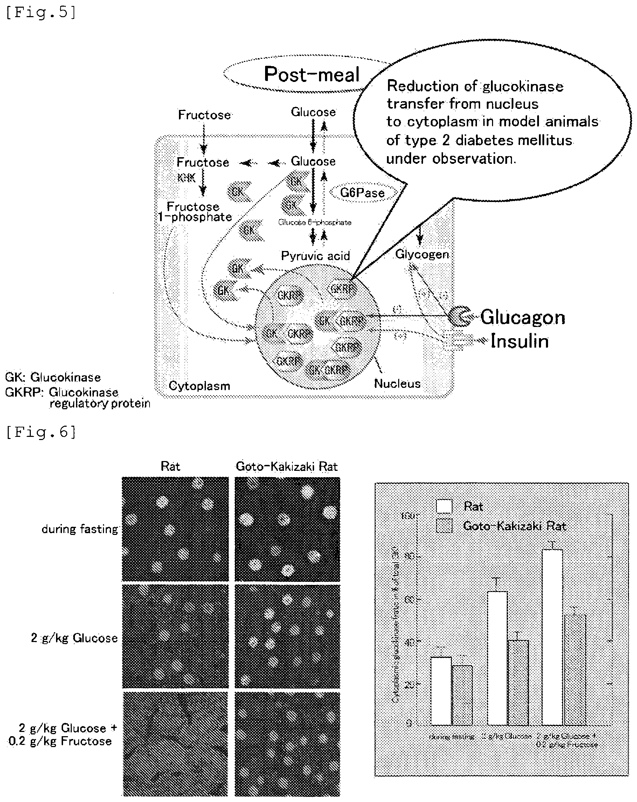

Fig. 5 shows a scheme describing the mechanism of the sugar metabolism in liver via the glucokinase transfer between nucleus and cytoplasm.

The inventors proposed "the existence of a mechanism of the sugar metabolism in liver via the glucokinase transfer between nucleus and cytoplasm", on the basis of the results described above. The inventors absolutely revealed the existence by the research works thereafter. Regarding the "mechanism of the sugar metabolism in liver via the glucokinase transfer between nucleus and cytoplasm", glucokinase exists in the inactive type at a normal blood glucose level via binding to the glucokinase regulatory protein in the nucleus. Due to the post-meal increase of blood glucose or the existence of D-fructose, glucokinase is dissociated from the glucokinase regulatory protein so that glucokinase becomes the active type and is transferred from the nucleus to the cytoplasm, where the glucokinase promotes the D-glucose metabolism. When the blood glucose level is lowered subsequently, the glucokinase returns to the nucleus, where the glucokinase binds to the glucokinase regulatory protein to become the inactive type. Such regulation mechanism is proposed (Fig. 5).

Fig. 6 shows photographs and bar graphs showing the change of the glucokinase distribution in hepatocytes 30 minutes after the oral administration of D-glucose or D-fructose.

Because the deterioration of sugar utilization in liver and the enhancement of gluconeogenesis in liver are observed in patients with type 2 diabetes mellitus, it was suggested that glucokinase transfer from the nucleus to the cytoplasm might be deteriorated in patients with type 2 diabetes mellitus. The inventors examined hepatic glucokinase transfer from nucleus to cytoplasm in Goto-Kakizaki rats as a model animal of type 2 diabetes mellitus, after oral administration of D-glucose or D-fructose to the rats. After oral administration of glucose to 2 g/1 kg body weight or of D-glucose to 2 g/1 kg body weight + D-fructose to 0.2 g/1 kg body weight to the Goto-Kakizaki rats, the glucokinase distribution in hepatocytes 30 minutes after loading was assayed. (Fig. 6) In the Goto-Kakizaki rats, insufficient glucokinase transfer from the nucleus to the cytoplasm occurred. The results indicate a possibility that the impairment of glucokinase transfer from nucleus to cytoplasm may be responsible for the deterioration of hepatic sugar utilization and the escalation of hepatic gluconeogenesis in patients with type 2 diabetes mellitus.

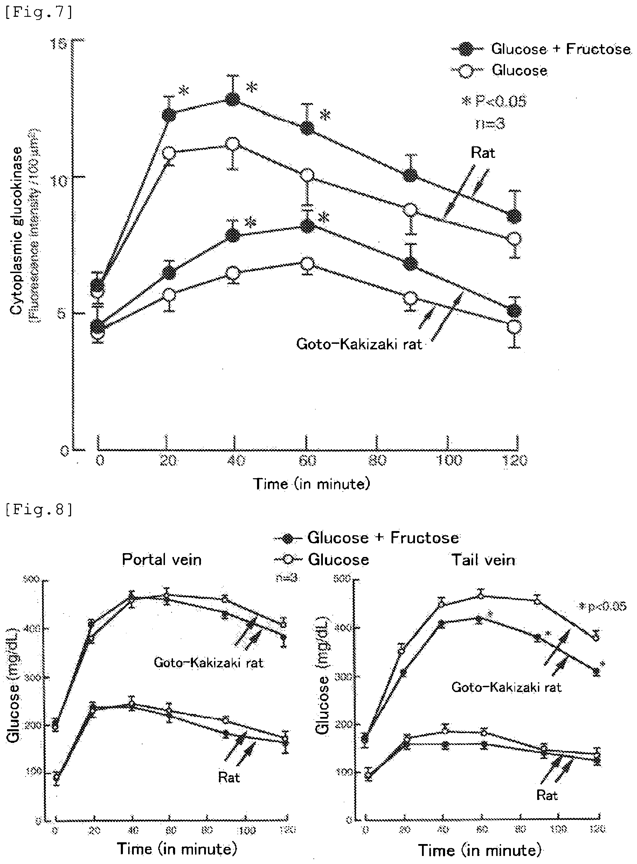

Fig. 7 shows graphs showing the action of suppressing blood glucose increase, via the change of the glucokinase distribution in hepatocytes with D-fructose at a low concentration.

The action of suppressing blood glucose increase with D-fructose at a low concentration was examined. To Goto-Kakizaki rats as a model animal of type 2 diabetes mellitus, D-glucose to 2 g /1 g body weight or D-glucose to 2 g/1 kg body weight + D-fructose to 0.2 g/ 1 kg body weight was administered orally, to examine the modification of glucokinase transfer from the nucleus to the cytoplasm in hepatocytes and the change of the blood glucose levels in the portal vein and the tail vein. Via the oral administration of D-glucose, glucokinase transferred from the nucleus to the cytoplasm. Via the addition of D-fructose, the glucokinase transfer from the nucleus to the cytoplasm was significantly increased, compared with the transfer during the administration of D-glucose alone.

Fig. 8 shows graphs showing the change of blood glucose levels after sugar loading.

Via sugar loading, the blood glucose level in the portal vein was increased (Fig. 8). No difference was observed due to the loading of D-fructose, but compared with the administration of D-glucose alone, the blood glucose level in the portal vein via the additional supplement of D-fructose was suppressed significantly. The suppression of the blood glucose level in the tail vein with D-fructose may be due to the increase of glucokinase transfer from the nucleus to the cytoplasm higher than the increase during the administration of D-glucose alone.

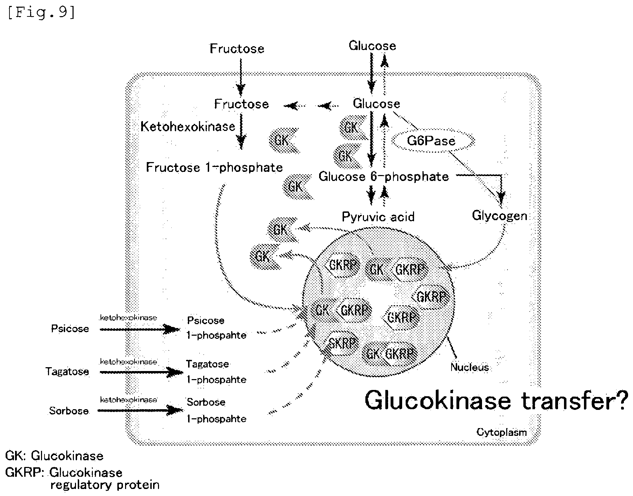

Fig. 9 is a scheme describing that a small amount of D-fructose administered can suppress the increase of blood glucose levels.

When a small amount of D-fructose is administered to patients with type 2 diabetes mellitus, the increase of the blood glucose levels can be suppressed (Fig. 9). This may be due to the occurrence of the glucokinase transfer from the nucleus to the cytoplasm in liver, leading to the promotion of sugar utilization in liver. This indicates that D-fructose may possibly be used as a sweetener for patients with diabetes mellitus, but excess intake of D-fructose may possibly induce the increase of blood lipids and uric acid, and the accumulation of lipids in liver. Therefore, interestingly, rare sugars may or may not have the same action as that of D-fructose.

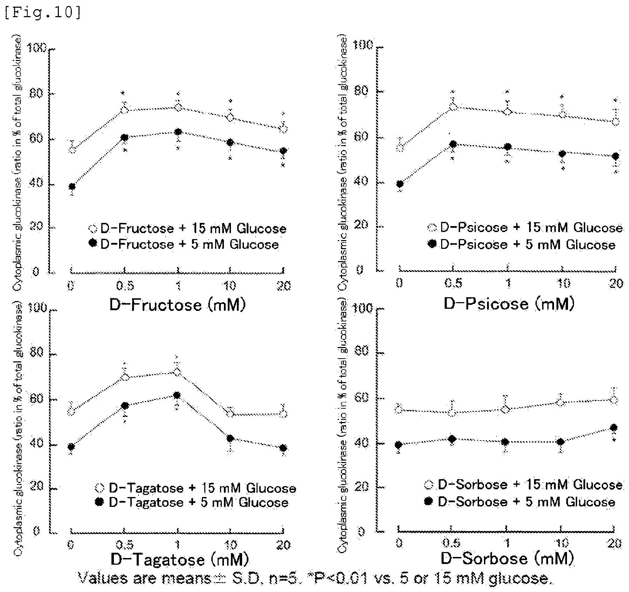

Fig. 10 shows graphs showing the effects of D-ketose on glucokinase transfer from nucleus to cytoplasm.

Hepatocytes were incubated in a D-ketose-supplemented MEM culture medium containing 5 mM or 15 mM glucose, to examine glucokinase transfer from nucleus to cytoplasm. The results are shown in Fig. 10.

In the presence of 5 mM or 15 mM D-glucose, glucokinase transferred from nucleus to cytoplasm via the addition of D-fructose. When D-fructose was added at 1 mM or more, however, glucokinase transfer from nucleus to cytoplasm was gradually reduced. At any of the D-glucose concentrations, glucokinase transferred from nucleus to cytoplasm on the addition of D-psicose. In case that D-tagatose was added up to 1 mM, glucokinase transferred from nucleus to cytoplasm at any of the D-glucose concentrations. When the D-tagatose concentration was 10 mM or more, no glucokinase transfer from nucleus to cytoplasm was observed. At any of the concentrations, D-sorbose hardly influenced such glucokinase transfer.

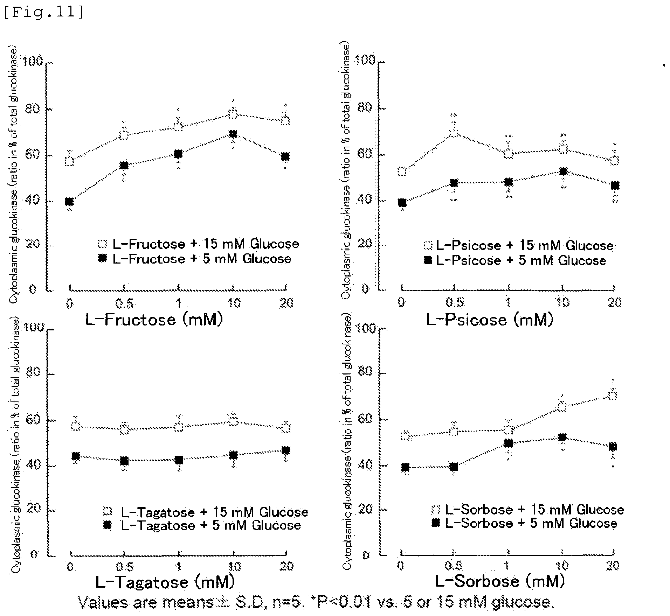

Fig. 11 shows graphs depicting the effects of L-ketose on glucokinase transfer from nucleus to cytoplasm.

Hepatocytes were incubated in an L-ketose-supplemented MEM culture medium containing 5 mM or 15 mM glucose, to examine glucokinase transfer from nucleus to cytoplasm. The results are shown in Fig. 11.

In the presence of 5 mM or 15 mM D-glucose, glucokinase transferred from nucleus to cytoplasm on the addition of L-fructose. When L-fructose was added at 20 mM or more, however, glucokinase transfer from nucleus to cytoplasm was gradually reduced. In the presence of D-glucose at 5 mM or 15 mM, glucokinase transferred from nucleus to cytoplasm via the addition of L-psicose. It was revealed that L-tagatose never influenced the glucokinase transfer. In case that L-sorbose was added, glucokinase transferred from nucleus to cytoplasm as the L-sorbose concentration was raised.

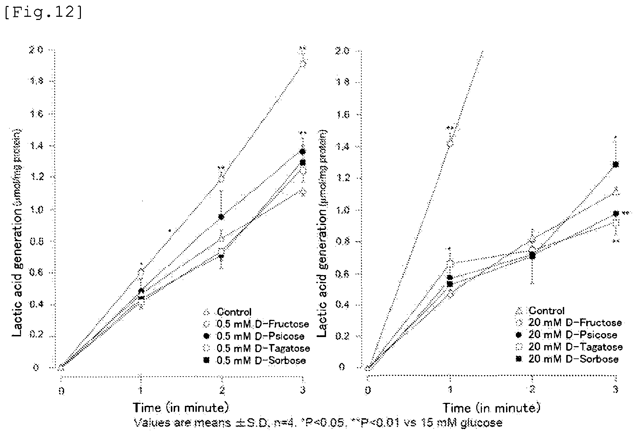

Fig. 12 shows graphs describing the effects of D-ketose on lactic acid generation.

Hepatocytes were incubated in a 5 mM or 20 mM D-ketose-supplemented MEM culture medium containing 15 mM glucose, to assay the amount of lactic acid in the MEM culture medium over time. As a control, DMSO was added. The results are shown in Fig. 12.

When each D-ketose was added at 0.5 mM, lactic acid generation was increased via the addition of D-fructose and D-psicose in the decreasing order.

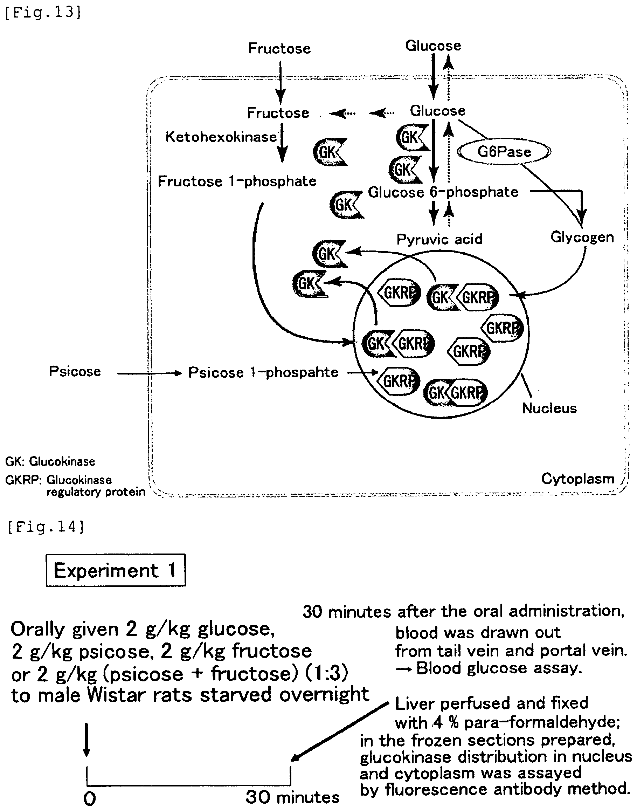

Fig. 13 is a scheme showing the effects of D-psicose on glucokinase transfer from nucleus to cytoplasm.

As shown in Fig. 10, D-psicose has an action of promoting glucokinase transfer from nucleus to cytoplasm. Therefore, the suppression of blood glucose increase through the action can be expected. Additionally, the same is true with D-tagatose at a low concentration.

Psicose is one of hexoses with a ketone group, among monosaccharides. It is known that psicose includes D form and L form as optical isomers. Herein, D-psicose is a known substance but exists rarely in the natural kingdom, so D-psicose is defined as "rare sugar" according to the definition of the International Society of Rare Sugars. D-Psicose for use in accordance with the invention is the D form of the psicose classified as ketose and is a hexose (C₆H₁₂O₆). Any D-psicose including D-psicose extracted in the natural kingdom and D-psicose synthetically prepared by chemical or biochemical synthetic methods may be available by any approaches. D-Psicose is enzymatically generated from D-fructose with an epimerase, which may be absolutely purified or may contain a trace amount of impurities during the enzymatic generation. Specifically, D-psicose is relatively readily prepared by an approach with an epimerase (see for example JP-A-6-125776). The resulting D-psicose solution is purified if necessary by methods such as deproteination, decoloring and desalting, and is then concentrated, from which a syrup-like D-psicose product can be collected. Via further fractionation and purification by column chromatography, a specimen at a purity as high as 99 % or more can readily be obtained. Such D-psicose can be used as it is as a monosaccharide.

Alternatively, L-psicose can be prepared from a sugar alcohol called allitol as a raw material, using a microorganism of the genus Gluconobacter, as described in JP-A-9-56390. Since the raw material allitol can be obtained readily via the reduction of D-psicose, allitol can advantageously be used for producing. L-psicose at a mass scale.

The bacterium for use in producing L-psicose from allitol belongs to the genus Gluconobacter, and is a bacterium with a potency to generate L-psicose from allitol. As one of the examples, Gluconobacter frauteri IFO3254 or a variant strain thereof may advantageously be used. Generally, such bacterium is cultured in a nutritious culture medium containing a carbon source such as glycerol, D-mannitol, D-fructose, L-sorbose or Xylitol, preferably under aerobic conditions such as shaking or aerated agitation, to convert allitol in the aqueous solution during culturing or using the resulting bacterium (fresh bacterial cell) to L-psicose. The generated L-psicose is satisfactorily collected.

The resulting aqueous L-psicose solution is purified if necessary by methods such as deproteination with desalting out with ammonium salt and adsorption with zinc hydroxide, decoloring with active charcoal adsorption, and desalting with type H or OH ion exchange resins, which is then concentrated. From the resulting concentrate is collected a syrup-like L-psicose product. By column chromatography with ion exchange resins for fractionation, purification and concentration, further, a specimen at a purity as high as 99 % or more can readily be obtained.

According to USP 5, 002, 612 and 5, 078, 796, D-tagatose is produced by using lactase for hydrolyzing lactose or a lactose-containing material into a mixture of D-galactose and D-glucose, from which D-glucose is optionally removed; subsequently, chemical isomerization of D-galactose to D-tagatose enables the production of D-tagatose. USP 6, 057, 135 describes D-tagatose production from cheese whey or milk, comprising hydrolyzing cheese whey or milk so as to prepare a mixture of galactose and glucose. D-Glucose is separated from D-galactose via D-glucose fermentation, which is then isomerized, using L-arabinose isomerase.

D-Psicose derivatives are now described. A compound with a modified molecular structure via a chemical reaction from a certain starting compound is called a derivative of the starting material. Derivatives of hexoses including D-psicose generally include sugar alcohols (aldehyde group and ketone group in a monosaccharide when reduced are converted to an alcohol group, so that a polyhydric alcohol with the same number of carbon atoms is generated), uronic acid (where the alcohol group in a monosaccharide is oxidized; as such uronic acid, there are known D-glucuronic acid, galacturonic acid, and mannuronic acid from natural origins), amino acids (where the OH group in a sugar molecule is substituted with NH₂ group; including glucosamine, chondrosamine and glycosides), but never limited to them. The same is true with derivatives of L-psicose and D-tagatose.

Further disclosed are foods additives, food materials, drinks and foods, health drinks and foods, pharmaceutical products and feeds, which can be used for preventing for example impaired glucose tolerance, type 2 diabetes mellitus, hyperlipidemia, the metabolic syndrome, the metabolic syndrome, and obesity, which are diseases in association with glucokinase activity, where the disordered conditions or the onset of such diseases can be ameliorated or prevented by promoting glucokinase transfer from nucleus to cytoplasm. The composition as a subject of the invention includes those for food additives, foods, healthy foods, foods for patients, food materials, healthy food materials, food materials for patients, food additives, healthy food additives, food additives for patients, drinks, healthy drinks, drinks for patients, drinking water, healthy drinking water, drinking water for patients, pharmaceutical products, raw materials for pharmaceutical preparations, feeds, and feeds for patient cattle and patient animals, which contain the composition in blend with D-psicose or D-psicose and D-tagatose as the active ingredient.

The invention relates to a promoting agent (pharmaceutical preparation) of glucokinase transfer from nucleus to cytoplasm, the promoting agent containing as the active ingredients D-psicose or D-psicose and D-tagatose. Additionally, the invention relates to drinks and foods with a label telling the efficacy thereof for use in preventing the onset of disordered conditions in association with glucokinase activity (disordered conditions selected from impaired glucose tolerance, type 2 diabetes mellitus, hyperlipidemia, the metabolic syndrome and obesity) and therapeutically treating the disordered conditions. Therefore, the pharmaceutical preparation or a food with health claims as the subject of the invention characteristically contains D-psicose or D-psicose and D-tagatose in blend as the active ingredient.

In case that D-psicose or D-psicose and D-tagatose is used as a pharmaceutical preparation, the pharmaceutical preparation can be prepared by known methods. Specifically, for example, the pharmaceutical preparation can be prepared as described below.

The pharmaceutical preparation of the invention is preferably used orally in forms of tablets coated with sugar coatings or other coatings, if necessary, pills, capsules (including hard capsules, soft capsules and micro-capsules), powders, granules, fine granules, troches, and liquids (including syrups, emulsions and suspensions).

The pharmaceutical preparation of the invention may be blended with physiologically acceptable carriers as long as the physiologically acceptable carriers never inhibit the advantages of the invention. As the physiologically acceptable carriers, there may be used various organic or inorganic carrier substances for routine use as materials for pharmaceutical preparations, including excipients, binders, disintegrators and lubricants for solid pharmaceutical preparations; and solvents, auxiliary dissolution agents, suspending agents, buffers, thickeners and emulsifiers for liquid pharmaceutical preparations. If necessary, further, additives for pharmaceutical preparations such as coloring agents, sweeteners and anti-oxidants, may be used. The pharmaceutical preparation of the invention may additionally be coated.

The excipients include for example lactose, purified sugar, D-mannitol, D-sorbitol, starch, α-starch, dextrin, crystalline cellulose (for example, micro-crystalline cellulose), hydroxypropylcellulose at a low substitution degree, carboxymethylcellulose sodium, gum Arabic, dextrin, pullulan, light silicic anhydride, synthetic aluminium silicate, and magnesium aluminometasilicate. The binders include for example α-starch, sucrose, gelatin, macrogol, gum Arabic, methylcellulose, carboxymethylcellulose, carboxymethylcellulose sodium, crystalline cellulose, purified sugar, D-mannitol, trehalose, dextrin, pullulan, hydroxypropylcellulose (HPC), hydroxypropyl methylcellulose (HPMC) and polyvinyl pyrrolidone (PVP). The disintegrators include for example lactose, purified sugar, starch, carboxymethylcellulose, carboxymethylcellulose calcium, crosslinked polyvinyl pyrrolidone, carmellose sodium, cross-carmellose sodium, sodium carboxymethyl starch, light silicic anhydride, hydroxypropylcellulose at a low substitution degree, cation exchange resins, partially gelatinized (α-) starch, and corn starch. The lubricants include for example stearic acid, magnesium stearate, calcium stearate, talc, waxes, colloidal silica, DL-leucine, sodium lauryl sulfate, magnesium lauryl sulfate, macrogol, and Aerosil.

The solvents include for example water for injections, physiological saline, the Ringer's solution, alcohols, propylene glycol, polyethylene glycol, medium-chain fatty acid triglyceride (MCT), vegetable oils (for example, safflower oil, sesame seed oil, corn oil, olive oil, cotton seed oil, soybean lecithin, etc.). The auxiliary dissolution agents include for example polyethylene glycol, propylene glycol, D-mannitol, trehalose, benzyl benzoate, ethanol, Tris-aminomethane, cholesterol, triethanolamine, sodium carbonate, sodium citrate, sodium salicylate, and sodium acetate. The suspending agents include for example detergents such as stearyl triethanolamine, sodium lauryl sulfate, laurylaminopropionic acid, lecithin, benzalkonium chloride, benzethonium chloride, and glycerin monostearate; hydrophilic polymers such as polyvinyl alcohol, polyvinyl pyrrolidone, carboxymethylcellulose sodium, methylcellulose, hydroxymethylcellulose, hydroxyethylcellulose, and hydroxypropylcellulose; and polysorbates and polyoxyethylene-hardened castor oil. The buffering agents include for example phosphate salts, acetate salts, carbonate salts, and citrate salts. The thickeners include for example natural rubbers and cellulose derivatives. The emulsifiers include for example fatty acid esters (for example, sucrose fatty acid ester, glycerin fatty acid ester, sorbitan fatty acid ester, propylene glycol fatty acid ester, etc.), waxes (for example, bee wax, hydrogenated rapeseed oil, hydrogenated safflower oil, hydrogenated palm oil, sitosterol, stigmasterol, canpesterol, brassica sterol, cacao fat powder, carnauba wax, rice wax, wood wax, paraffin, etc.), and lecithin (for example, egg yolk lecithin, soybean lecithin, etc).

The coloring agents include for example water-soluble edible tar dyes (for example, edible dyes such as edible Red No.2 and No.3, edible Yellow No. 4 and No.5, and edible Blue No.1 and No.2), water-insoluble lake dyes (for example, the water-soluble edible tar dyes in aluminium salts), and natural dyes (for example, β-carotene, chlorophyll, bengara, etc.). The sweetener includes for example sucrose, lactose, sodium saccharin, dipotassium glycyrrhizinate, aspartame, and stevia. The anti-oxidants include for example sulfite salts, ascorbic acid and alkali metal salts and alkali earth metal salts thereof.

For the purpose of taste masking, photo-stability improvement, appearance improvement and enteric coating, for example, the tablets, the granules and the fine granules may be coated, using coating materials. The coating base materials include for example sugar coating base materials, water-soluble film coating base materials, and enteric film coating base materials.

The sugar coating base materials include for example purified sugar, satisfactorily in combination with one or two or more selected from talc, precipitated calcium carbonate, gelatin, gum Arabic, pullulan, and carnauba wax.

The water-soluble film coating base materials include for example cellulose-series polymers such as hydroxypropylcellulose (HPC), hydroxypropylmethylcellulose (HPMC), ethylcellulose, hydroxyethylcellulose, and methyl hydroxyethylcellulose; synthetic polymers such as polyvinyl acetal diethylaminoacetate, aminoalkylmethacrylate copolymer E (Eudragit under the trade name as manufactured by Rohm Pharm) polyvinyl pyrrolidone; and polysaccharides such as pullulan. The enteric film coating base materials include for example cellulose-series polymers such as hydroxypropylmethylcellulose phthalate, hydroxypropylmethylcellulose acetate succinate, carboxymethylethylcellulose, and cellulose acetophthalate; acrylic acid-series polymers such as methacrylate copolymer L [Eudragit L (under the trade name) Rohm Pharm], methacrylate copolymer LD [Eudragit L -30D 55 (under the trade name) Rohm Pharm], methacrylate copolymer S [Eudragit S (under the trade name) Rohm Pharm] ; and natural products such as Shellac. These coating base materials may be used singly or two or more thereof may be mixed together at an appropriate ratio for use in coating. Furthermore, two or more thereof may sequentially be used for coating.

In case of a composition in a form of a drink or a food or a drink or a food with a label telling the functions and efficacies, the food composition or drink or food in accordance with the invention may be prepared as a functional food, a health food, a food with health claims, a food for patients, or a supplement. Preferably, the food composition or drink or food in accordance with the invention may be prepared as a functional food. The form of such food composition or drink or food includes for example tablets, pills, capsules (hard capsules, soft capsules, and micro-capsules), powders, granules, fine granules, troches, liquids (including syrups, emulsions and suspensions). Preferably, the form thereof is a table or a capsule.

The food composition is particularly preferably a tablet or a capsule. In particular, the food composition is preferably a functional food in tablets or a functional food in capsules.

The food composition can be produced by allowing a food to contain D-psicose or D-psicose and D-tagatose by known methods. Specifically, for example, the food composition in a tablet can be prepared by adding and mixing together raw materials such as D-psicose or D-psicose and D-tagatose, and excipients (for example, lactose, purified sugar, and mannitol), sweeteners and flavor and then pressurizing the resulting mixture with a tableting machine and the like to mold the mixture into a tablet. If necessary, other materials (for example, vitamins such as vitamin C, minerals such as iron, and dietary fiber) may be added. The food composition in capsules can be produced by filling a food composition containing D-psicose or D-psicose and D-tagatose in liquids, suspensions, pastes, powders or granules into a capsule or by enveloping and molding the food composition with a capsule base material.

The food composition may be blended with physiologically acceptable carriers in addition to food materials, food additives, various nutrients, vitamins, and flavor substances (for example, cheese and chocolate) for routine use, as long as they never inhibit the advantages of the invention. As the physiologically acceptable carriers, various organic or inorganic carrier substances for routine use may be used and include for example excipients, binders, disintegrators, lubricants, colorants, sweeteners, preservatives, anti-oxidants, thickeners, and emulsifying agents. Additionally, the food additives include for example colorants, sweeteners, preservatives, anti-oxidants and flavor. Furthermore, the food composition of the invention may satisfactorily contain other materials for example minerals such as iron, and dietary fibers such as pectin, carrageen, and mannan.

As the excipients, the binders, the disintegrators, the lubricants, the solvents, the auxiliary dissolution agents, the suspending agents, the buffer agents, the thickeners, the colorants, the sweeteners, the preservatives and the anti-oxidants, those described for use in the pharmaceutical preparation of the invention may also be listed.

The vitamins may be water-soluble or fat-soluble and include for example retinol palmitate, tocopherol, bisbenthiamine, riboflavin, pyridoxine hydrochloride, cyanocobalamin, sodium ascorbate, cholecalciferol, nicotinate amide, calcium pantothenate, folic acid, biotin and choline bitartrate.

As to the food composition in tablets, granules and fine granules, coating base materials may be used for coating by known methods for the purpose of taste masking, photo-stability improvement, appearance improvement or enteric coating. As the coating base materials, the same ones as those for use in the pharmaceutical preparation of the invention may be listed. Coating may be done in the same manner.

The composition of the invention is a composition containing D-psicose or D-psicose and D-tagatose together with one or more pharmaceutically acceptable carriers for use in preventing the onset of disordered conditions in association with glucokinase activity and therapeutically treating the disordered conditions, and the composition of the invention is in the form of a pharmaceutical product. The pharmaceutical composition of the invention is suitable for enteral administrations for example oral or trans-rectal administration, trans-dermal administration or parenteral administration to mammals including humans so as to promote and activate glucokinase transfer from nucleus to cytoplasm, and is suitable for treatment of disordered conditions in association with glucokinase activity. Such disordered conditions include
impaired glucose tolerance, type 2 diabetes mellitus, hyperlipidemia, the metabolic syndrome, the metabolic syndrome and obesity as described above. The pharmaceutical composition may be used singly or in combination with one or more pharmaceutically acceptable carriers and contains a therapeutically effective amount of the pharmacologically active compound.

The pharmacologically active compound of the invention is useful for producing a pharmaceutical composition containing a therapeutically effective amount of the pharmacologically active compound in association or in mixture with excipients or carriers suitable for enteral or parenteral administration. Diluents for example lactose, dextrose, sucrose, mannitol, sorbitol, cellulose and/or glycine; lubricants for example silica, talcum, stearic acid, the magnesium salt thereof or calcium salt thereof and/or polyethylene glycol are suitable. For tablets, additionally, binders for example magnesium aluminosilicate, starch paste, gelatin, gum tragacanth, methylcellulose, sodium carboxymethylcellulose, and/or polyvinyl pyrrolidone; disintegrators for example starch, agar, alginic acid or the sodium salt thereof; or foaming mixtures and/or absorbing agents; colorants; flavor and sweeteners may be contained together with the active ingredient in a tablet or a gelatin capsule, which is preferable.

The composition for injections is preferably an aqueous isotonic solution or a suspension, while suppositories are produced from fat emulsions or suspensions, advantageously. Such composition is sterilized and/or may contain adjuvants for example preservatives, stabilizers, moistening agents or emulsifiers, solution promoters, osmotic pressure-adjusting salts and/or buffering agents. Furthermore, they may contain other therapeutically valuable substances. The composition is produced individually by routine mixing, granulation or coating methods, and contains the active ingredient at about 0.1 to 75 %, preferably about 1 to 50 %.

The pharmaceutical preparation suitable for trans-dermal administration contains a therapeutically effective amount of the compound of the invention, along with carriers. Advantageous carriers include pharmacologically acceptable solvents absorbable, which help the passage through host skins. Characteristically, the trans-dermal device is in a form including a backing member, a reservoir containing the compound together with carriers, if desired, a rate control barrier delivering the compound at a predetermined controlled rate for a long term to host skins, and a bandage including units for fixing the device on the skin.

The pharmaceutical preparation contains a therapeutically effective amount of the compound of the invention as described above, singly or in combination with other therapeutic agents for example at individually therapeutically effective amounts reported in the field. Such therapeutic agents include for example insulin, insulin derivatives or mimetic thereof; insulin secretion-promoting agents; insulin secretion-enhancing sulfonyl urea receptor ligands; PPAR ligands; insulin sensitizer; biguanide, alpha-glucosidase inhibitors; GLP-1, GLP-1 analogs or mimetics thereof; DPPIV inhibitors; HMG-CoA reductase inhibitors; squalene synthase inhibitors; FXR or LXR ligands; cholestyramine; fibrate; nicotinic acid or aspirin.

In an additional embodiment, therefore, the invention relates to a pharmaceutical composition containing a therapeutically effective amount of the compound of the invention in combination with one or more pharmaceutical carriers.

In an additional embodiment, the invention relates to a pharmaceutical composition containing a therapeutically effective amount of the compound of the invention in combination with other therapeutic agents at therapeutically effective amounts thereof, which are selected preferably from anti-diabetic agents, lipid-reducing agents, anti-obesity agents, hypotensive agents or cardiotonic agents, most preferably from the anti-diabetic agents or the lipid-reducing agents.

The invention relates to a pharmaceutical composition or a combined agent therewith for producing pharmaceutical agents for treating disordered conditions in association with glucokinase activity, more specifically impaired glucose tolerance, type 2 diabetes mellitus, hyperlipidemia, metabolic syndrome, the metabolic syndrome and obesity, preferably type 2 diabetes mellitus, impaired glucose tolerance and obesity.

The invention relates to the pharmaceutical composition for therapeutically treating disordered conditions in association with glucokinase activity, i.e., impaired glucose tolerance, type 2 diabetes mellitus, hyperlipidemia, the metabolic syndrome, the metabolic syndrome and obesity.

The unit dose for mammals of a body weight of about 50 to 70 kg may contain the active ingredient of about 1 mg to 20,000 mg, advantageously about 5 mg to 5,000 mg. The therapeutically effective amount of the compound depends on the species of a warm-blooded animal (mammalians), and the body weight, age and individual conditions thereof, and the form of dosing and compounds in relation.

The D-psicose or D-psicose and D-tagatose as the compound of the invention is a promoting agent of the glucokinase transfer from the nucleus to the cytoplasm. Thus, the compound of the invention is used for treating disordered conditions in association with glucokinase activity as described above, i.e., impaired glucose tolerance, type 2 diabetes mellitus, hyperlipidemia, the metabolic syndrome, the metabolic syndrome and obesity.

According to those described above, the invention provides those described below in an additional aspect. The invention relates to a therapeutic combination agent for use in any of the methods, for example a kit or a part kit containing the compound of the formula I in the free form or in a pharmaceutically acceptable salt form, for simultaneous use or sequential use with at least one pharmaceutical composition containing at least one of other therapeutic agents (preferably, anti-diabetic agents, lipid-reducing agents, anti-obesity agents, hypotensive agents or cardiotonic agents). The kit may contain instruction manuals for the administration. The kit comprises parts comprising pharmaceutical compositions in the individual two units of the constitution elements (i) and (ii), namely (i) the pharmaceutical composition of the invention and (ii) a compound selected from anti-diabetic agents, anti-obesity agents, hypotensive agents, cardiotonic agents or lipid-reducing agents, or pharmaceutically acceptable salts thereof.

The compound of the invention is preferably administered to mammals needing the compound.

Preferably, the compound of the invention is used for treating diseases in response to the activation of glucokinase activity.

The disordered conditions in association with glucokinase activity are selected from impaired glucose tolerance, type 2 diabetes mellitus, hyperlipidemia, the metabolic syndrome and obesity, and preferably selected from type 2 diabetes mellitus, impaired glucose tolerance and obesity.

The method of the invention and the use thereof, comprising the administration of the compound in combination with a therapeutically effective amount of anti-diabetic agents, anti-obesity agents, hypotensive agents, cardiotonic agents or lipid-reducing agents.

The method of the invention and the use thereof, comprising the administration of the compound in the form of a pharmaceutical composition described in this specification.

As used throughout the specification and in the claims, the term "treating" means treatment in all of various forms and modes known to persons skilled in the art, and particularly includes prophylactic and curative treatment, and treatment for the delay of the progress and for the mitigation.

The characteristic profiles are advantageously exerted in tests in vitro and in vivo using mammals, for example, mouse, rat, dog, monkey, resected organs, tissues and prepared products therefrom. The compound is applicable in vitro in solutions, preferably for example aqueous solutions, and is also applicable in vivo enterally, parenterally, advantageously transvenously or in forms of for example suspensions or aqueous solutions. The dose in vitro is within a range of about 10⁻² molar concentration to 10⁻⁶ molar concentration. The therapeutically effective dose in vivo depends on the dosage route, which may be in a range of about 0.1 to 20,000 mg/kg, preferably about 1 to 5,000 mg/kg.

The details of the invention will now be described in Examples. The invention is never limited by these Examples.

### Example 1

Liver is an important organ for the maintenance of the homeostasis of blood glucose. One of the rate limiting enzymes of glycolysis in the sugar metabolism in liver is glucokinase (EC 2.7.1.1) existing in the inactive type via the binding to the glucokinase regulatory protein in the cell nucleus during fasting. Due to the increase of extracellular D-glucose concentration and the existence of D-fructose at a low concentration, glucokinase is dissociated from the glucokinase regulatory protein and is transferred from the nucleus to the cytoplasm. In such manner, glucokinase promotes D-glucose metabolism. Glucokinase transfer between the nucleus and the cytoplasm is regulated by hormones. Insulin transfers glucokinase from the nucleus to the cytoplasm while glucagon transfers glucokinase from the cytoplasm to the nucleus.

In recent years, reports have bee issued about a glucokinase-activating agent with a hypoglycemic action via the enhancement of glucokinase activity. Additionally, a method for producing rare sugars at a mass scale has been developed, so attention has now been focused on the functions of rare sugars.

In the present research works, a signal transduction system about hepatic glucokinase transfer between nucleus and cytoplasm with insulin and glucagons was examined for the purpose of developing a therapeutic agent of diabetes mellitus via the promotion of hepatic glucokinase transfer from nucleus to cytoplasm. For the purpose of screening for a sugar replacing D-fructose, furthermore, ketose influence on the glucokinase transfer between nucleus and cytoplasm was examined.

### [Experimental methods]

### 1. Intracellular transfer of hepatic glucokinase with hormones

Wistar rats (male, aged 5 to 8 weeks) were used. A primary culture of the rat hepatocyte was incubated for a certain period of time in a 25 mM D-glucose-containing MEM culture medium supplemented with hormones and various compounds. The hepatocytes were fixed with 4 % para-formaldehyde, and stained by a fluorescence antibody method using an anti-glucokinase antibody. A fluorescent image obtained with a co-focus laser microscope was analyzed with the NIH Image to calculate the fluorescence intensity in the nucleus and the cytoplasm, which were defined as the glucokinase level in the nucleus and the cytoplasm.

### 2. Intracellular transfer of hepatic glucokinase with ketose

The rat hepatocyte primary culture was incubated for a certain period of time in a ketose-added MEM culture medium containing 5 mM or 15 mM D-glucose. By the same method as described above, the glucokinase level in the intracellular nucleus and the cytoplasm was determined.

### [Results and Discussion]

### 1. Hormonal regulation of intracellular transfer of rat hepatocyte glucokinase

In the presence of insulin, LY-294002 was allowed to exert its action. The increase of cytoplasmic glucokinase with insulin was reduced. In the presence of insulin, PD-98059 or rapamycin was allowed to exert its action. In any of the cases, consequently, such change could not be observed. Forskolin, Bt₂cAMP and IBMX were allowed to exert their actions. In any of the cases, cytoplasmic glucokinase activity was reduced as in the case of glucagon addition. KT-5720 was allowed to exert its action in the presence of glucagon. Almost no reduction of cytoplasmic glucokinase with glucagon was observed any more. It is considered that glucokinase transfer from nucleus to cytoplasm with insulin might be mediated through PI3 kinase, while glucokinase transfer from cytoplasm to nucleus might be mediated through the cAMP dependent kinase.

### 2. Glucose promotion of glucokinase transfer from nucleus to cytoplasm

Cultured hepatocytes were incubated in an MEM culture medium containing 5 mM or 25 mM D-glucose with an action of promoting glucokinase transfer from nucleus to cytoplasm. Glucokinase transfer from nucleus to cytoplasm, glycogen synthesis and lactic acid generation were increased. Via glucose, glucokinase transfer from nucleus to cytoplasm occurs, which raises cytoplasmic glucokinase activity to possibly promote the sugar metabolism in liver. (Graphs showing glucose administration in Figs.2, 4 and 6) It was revealed that a trace amount of D-fructose promoted glucokinase transfer from nucleus to cytoplasm. (Figs. 4, 6, 7 and 8) 3. Ketose promotion of glucokinase transfer from nucleus to cytoplasm

When D-Fructose, D-psicose or D-tagatose was added individually, glucokinase transferred from nucleus to cytoplasm in the presence of 5 mM or 15 mM D-glucose. In case that D-tagatose was added at 10 mM or more, no change of the glucokinase transfer was observed. At any of the concentration, D-sorbose did not influence glucokinase transfer. Even at a high concentration, D-psicose exerted an action to promote glucokinase transfer from nucleus to cytoplasm. (Fig. 11)

### [Conclusion]

1. Glucokinase transfer from nucleus to cytoplasm with insulin occurs through PI3 kinase, while glucokinase transfer from cytoplasm to nucleus with glucagon occurs through the cAMP-A kinase.
2. When glucokinase was incubated with a primary culture of hepatocytes, glucokinase transfer from nucleus to cytoplasm occurs, to increase the glycogen level and the lactic acid generation.
3. With psicose and tagatose at a low concentration, glucokinase transfer from nucleus to cytoplasm occurs.

### Example 2

Experiments were carried out to verify whether or not glucokinase transfer from nucleus to cytoplasm as observed in the culture cell occurred even in animal experiments.

### [Experimental methods]

Fig. 14 shows the protocols. Male Wistar rats starved overnight were orally given D-glucose, D-psicose, D-fructose and a mixture of D-psicose and D-fructose at 1: 3, at individual doses of 2 g/kg. Thirty minutes later, the rats were subjected to perfusion and fixing with 4 % para-formaldehyde under anesthesia, from which liver was immediately resected to prepare frozen sections. The distribution of glucokinase in nucleus and cytoplasm was analyzed with the fluorescence antibody method using an anti-glucokinase antibody. Simultaneously, blood was drawn out from the tail vein and the portal vein, to assay blood glucose levels.

### [Results and discussion]

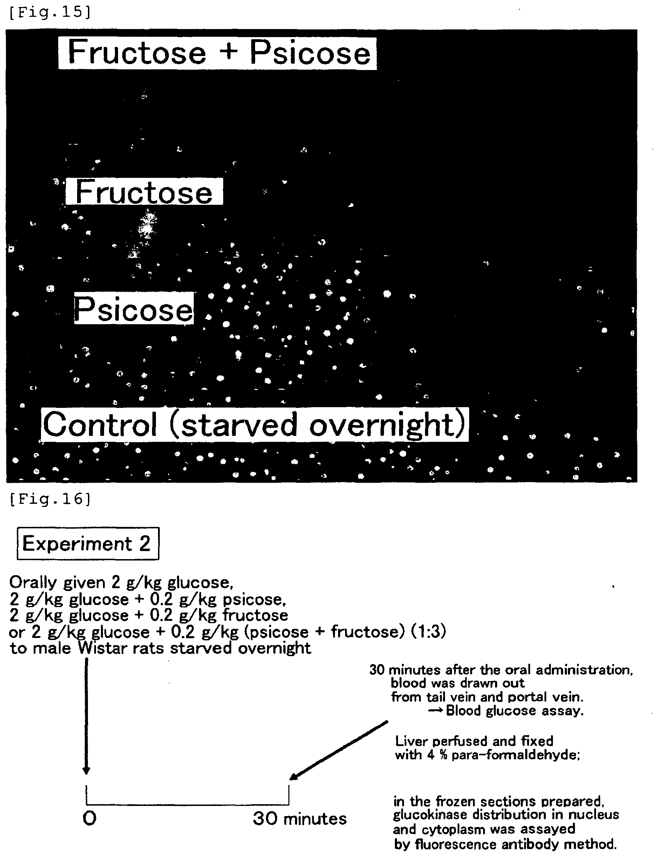

Fig.15 shows the results.

In the control (starved overnight), glucokinase mostly exists in the hepatocyte nucleus. When D-psicose was administered, glucokinase transfer from the nucleus to the cytoplasm could be observed in the hepatocytes on the right side of the photographs. With D-fructose, a higher level of the transfer was observed than with D-psicose. Even with the mixture of D-fructose and D-psicose, sufficient glucokinase transfer from the nucleus to the cytoplasm was observed.

### [Conclusion]

1. The phenomenon of glucokinase transfer from nucleus to cytoplasm as observed in the culture cell was also verified at the experiments for animal administration.
2. At the experiments with the rats, it was revealed that D-fructose caused glucokinase transfer from the nucleus to the cytoplasm.
3. With D-psicose and also even with the mixture of D-fructose and D-psicose, such transfer was observed.

### Example 3

For the purpose of mimicking general states of meals, rats orally given with a given amount of D-glucose were simultaneously given D-psicose or D-fructose or a mixture of D-psicose and D-fructose at an amount 1/10-fold the given amount of D-glucose, to certify whether or not glucokinase transfer from nucleus to cytoplasm emerged experimentally.

### [Experimental methods]

Fig. 16 shows the protocols. Male Wistar rats starved overnight were given 2 g/kg D-glucose orally. Simultaneously, 0.2 g/kg D-psicose, D-fructose or a mixture of D-psicose and D-fructose at 1: 3 were given orally. Thirty minutes later, the rats were perfused and fixed with 4 % para-formaldehyde under anesthesia, from which liver was immediately resected to prepare frozen sections. The distribution of glucokinase in the nucleus and the cytoplasm was analyzed with the fluorescence antibody method using an anti-glucokinase antibody. Simultaneously, blood was drawn out from the tail vein and the portal vein, to assay blood glucose levels.

### [Results and discussion]

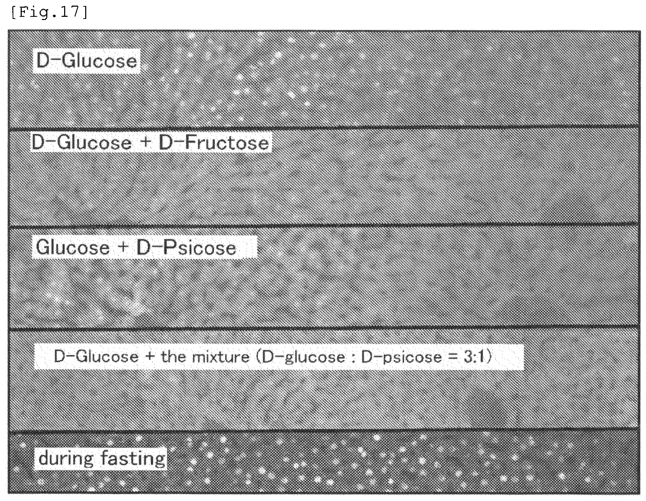

Fig.17 shows the results.

In the control (marked with "Fasted" in the lowest bottom), glucokinase mostly exists in the hepatocyte nucleus.

At the oral administration of 2 g/kg D-glucose (the uppermost column), glucokinase transfer from the nucleus to the cytoplasm occurred slightly (the transfer was at higher levels in the cell groups on the right side, in particular) .

At the oral administration of 0.2 g/kg D-fructose simultaneously with the D-glucose administration (on the upper second column), glucokinase was mostly transferred to the cytoplasm. At the oral administration of 0.2 g/kg D-psicose simultaneously with the D-glucose administration (on the upper third column), glucokinase was transferred to the cytoplasm apparently but more weakly than with D-fructose (the transfer was frequently observed in the cell groups on the right side, in particular) . With the mixture of D-fructose and D-psicose (on the upper fourth column), glucokinase transfer from the nucleus to the cytoplasm occurred more strongly than the case administered with D-fructose alone. In a near future, it will be elucidated whether or not the transfer to the cytoplasm with D-tagatose as observed in the cell experiments would occur.

### [Conclusion]

1. It was revealed at the experiments in the rat that glucokinase transfer from nucleus to cytoplasm was promoted by administering D-fructose and D-psicose simultaneously with D-glucose.
2. The promotion was the highest with the mixture of D-fructose and D-psicose. The promotion was then decreased in the order of D-fructose and D-psicose. Even with D-psicose alone, such transfer was apparently observed.
3. Glucokinase transfer from the nucleus to the cytoplasm was promoted by ingestion of the mixture of D-fructose and D-psicose or of D-psicose alone, together with meals. Glycogen synthesis was promoted due to the mechanisms described in Figs. 1 and 5, so that blood D-glucose transferred intracellularly, consequently leading to a possible reduction of blood glucose level.

### Example 4

So as to examine whether or not D-psicose had an action of ameliorating the disordered conditions of patients with type 2 diabetes mellitus, patients with IGT (impaired glucose tolerance, a provisional group of diabetes mellitus) and patients with the metabolic syndrome, D-psicose was orally administered to Goto-Kakizaki rats as a model animal of type 2 diabetes mellitus, to assay the hepatic glucokinase transfer from the nucleus to the cytoplasm, and blood glucose levels and insulin levels in the tail vein and the portal vein.

### [Experimental methods]

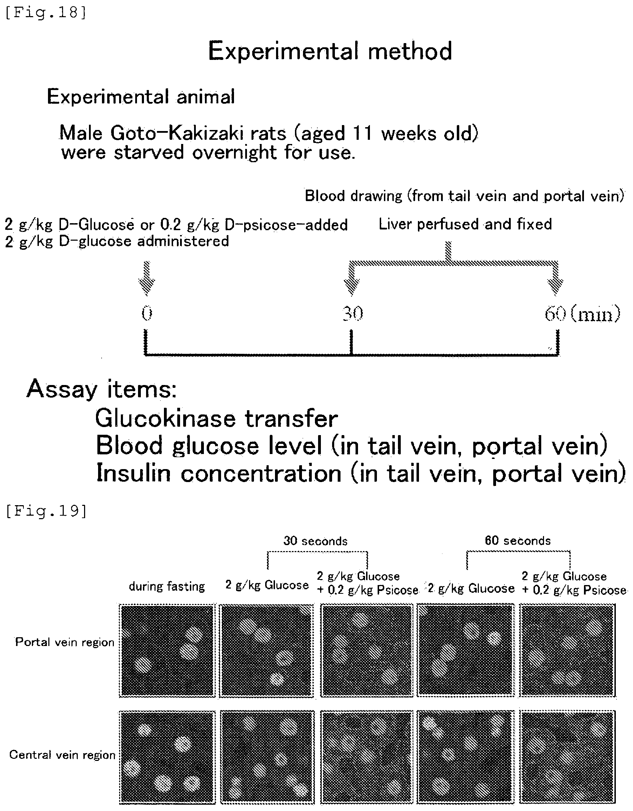

Fig.18 shows the protocols. 2 g/kg D-glucose is orally given to male Goto-Kakizaki rats starved overnight. Simultaneously, 0.2 g/kg D-psicose was orally given. Thirty minutes later and 60 minutes later, the rats were perfused and fixed with 4 % para-formaldehyde under anesthesia, from which liver was immediately resected to prepare frozen sections. The distribution of hepatic glucokinase in nucleus and cytoplasm was analyzed with the fluorescence antibody method using an anti-glucokinase antibody. Simultaneously, blood was drawn out from the tail vein and the portal vein, to assay blood glucose level and insulin concentration. Herein, liver is divided in the portal vein region and the central vein region of liver. It has been known that the activity of glucokinase in the central vein region is higher by 1.3 fold to 1.5 fold than the activity thereof in the portal vein region. Therefore, the glucokinase distributions in the nucleus and the cytoplasm in the central vein region and the portal vein region were analyzed.

### [Results and Discussion]

1. Fig.19 shows the assay results of the glucokinase distribution in the liver 30 minutes and 60 minutes after the administration of D-glucose alone or the simultaneous administration of D-glucose and D-psicose, by the fluorescence antibody method.
   Fluorescence specific to glucokinase was mainly observed in the nucleus in the Goto-Kakizaki rats starved for 24 hours. Fluorescence in the nucleus was reduced at any timing in the time course via the oral administration of D-glucose, while fluorescence in the cytoplasm was inversely increased. The simultaneous administration with D-psicose significantly reduced the nuclear fluorescence but increased the cytoplasmic fluorescence compared with the administration of D-glucose alone. The change was observed in both of the portal region and the central vein region.

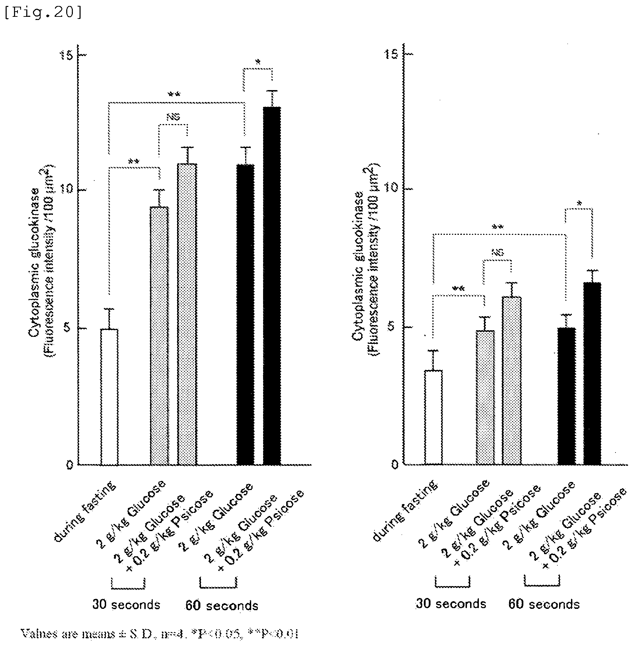

2. Fig. 20 shows the analytical results of the fluorescence intensity in the nucleus and the cytoplasm in the images in Fig. 19.
   In the central vein region and the portal vein region, the cytoplasmic glucokinase level was significantly increased via the oral administration of D-glucose than the glucose level at the 24-hour fasting. The simultaneous administration with D-psicose further increased the cytoplasmic glucokinase level. Under the present experimental conditions, no change of the glucose levels in total in the nucleus and the cytoplasm was observed. These results indicate that glucokinase transfer from the nucleus to the cytoplasm occurred in vivo with D-psicose, so that the active-type glucokinase was increased, leading to the occurrence of the promotion of sugar utilization in liver.

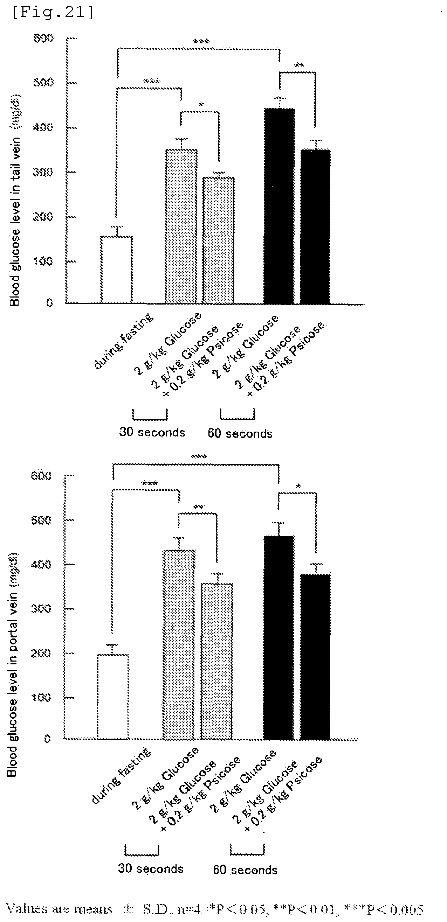

3. Fig.21 shows the change of the blood glucose levels in the tail vein and the portal vein.
   The glucose concentrations in the tail vein and the portal vein 30 minutes and 60 minutes after the simultaneous administration of D-psicose and D-glucose were at significantly lower values than the value in the group administered D-glucose alone.
   The results show that the active type glucokinase increased due to the glucokinase transfer from the nucleus to the cytoplasm with D-psicose, promoting sugar utilization in liver leading to the occurrence of the suppression of the increase of the blood glucose levels.

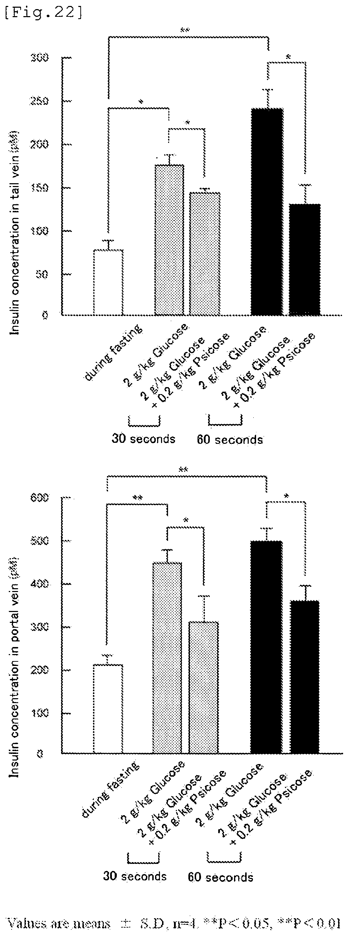

4. Fig.22 shows the change of the insulin concentrations in the tail vein and the portal vein under sugar loading.

The insulin concentrations in the tail vein and the portal vein 30 minutes and 60 minutes after the simultaneous administration of D-psicose and D-glucose were at significantly lower values than the values therein in the group administered with D-glucose alone.

The results show that due to the glucokinase transfer from the nucleus to the cytoplasm with D-psicose, sugar utilization in liver is promoted to suppress the increase of the blood glucose level, so that the suppression of insulin secretion from pancreatic β cells occurred. It is understood that the suppression of insulin secretion leads to the preservation of pancreatic β cells.

The aforementioned results indicate that D-psicose has an action of ameliorating the disordered conditions of patients with type 2 diabetes mellitus, patients with IGT (impaired glucose tolerance, a provisional group of diabetes mellitus) and patients with the metabolic syndrome. It is understood that D-tagatose has the same action as that of D-psicose.

### [Conclusion]

The oral administration of D-psicose simultaneously with the D-glucose administration to Goto-Kakizaki rats as a type 2 diabetes mellitus model animal more highly transfers hepatic glucokinase from the nucleus to the cytoplasm, compared with the administration of D-glucose alone, leading to the promotion of sugar utilization in liver to reduce the increase of the blood glucose level and the increase of the insulin concentration.

### Industrial Applicability

Since glucokinase is important not only for insulin secretion but also for the hypertrophy of compensated pancreatic β cells against insulin resistance with hyper fat diets, glucokinase or glucokinase-activating agents promote insulin secretion from pancreatic β cells and also allows for the increase of the amount of pancreatic β cells in disordered conditions with the endogenous genetic deposition of reduced insulin secretion along with the affliction of obesity with hyper fat diets and insulin resistance. Hence, insulin secretion potency is ameliorated in the disordered conditions. Therefore, glucokinase or glucokinase-activating agents are useful for a method for therapeutically treating type 2 diabetes mellitus. Particularly, D-psicose and D-tagatose are monosaccharides naturally occurring so such monosaccharides may have less adverse actions. Additionally, it is shown that D-fructose has an action of promoting glucokinase transfer from nucleus to cytoplasm. However, D-fructose has such energy that an excess intake of D-fructose is problematic, disadvantageously. Compared with D-fructose, D-psicose and D-tagatose have an action of promoting glucokinase transfer from nucleus to cytoplasm like D-fructose but have no energy, so D-psicose and D-tagatose are at a low possibility of causing the exacerbation of diabetes mellitus and hyperlipidemia. Accordingly, it is suggested that D-psicose and D-tagatose are highly efficacious.

## Claims

1. A composition containing as the active ingredients D-psicose or D-psicose and D-tagatose for use in preventing the onset of and therapeutically treating disordered conditions in association with glucokinase activity selected from impaired glucose tolerance, type 2 diabetes mellitus, hyperlipidemia, the metabolic syndrome and obesity.

2. The composition for use in preventing the onset of and therapeutically treating disordered conditions in association with glucokinase activity according to claim 1, wherein the composition is in a form selected from the group consisting of food additives, food materials, foods and drinks, health foods and drinks, pharmaceutical products and feeds in blend with D-psicose or D-psicose and D-tagatose as the active ingredient.

3. The composition for use in preventing the onset of and therapeutically treating disordered conditions in association with glucokinase activity according to claim 2, wherein the composition is in a form of pharmaceutical product and contains D-psicose or D-psicose and D-tagatose together with one or more pharmaceutically acceptable carriers.

4. The composition for use in preventing the onset of disordered conditions in association with glucokinase activity and therapeutically treating the disordered conditions according to any one of claims 1 to 3, wherein the amount of D-psicose or D-psicose and D-tagatose as the effective amount thereof is 0.5 to 50 g daily.

## Patentansprüche

1. Zusammensetzung, die als die aktiven Inhaltsstoffe D-Psicose oder D-Psicose und D-Tagatose enthält, für die Verwendung in der Vermeidung des Einsetzens und der therapeutischen Behandlung von gestörten Zuständen in Assoziation mit Glucokinaseaktivität, ausgewählt aus beeinträchtigter Glucosetoleranz, Typ 2 Diabetes mellitus, Hyperlipidämie, dem metabolischen Syndrom und Adipositas.

2. Zusammensetzung für die Verwendung in der Vermeidung des Einsetzens und der therapeutischen Behandlung von gestörten Zuständen in Assoziation mit der Glucokinaseaktivität nach Anspruch 1, wobei die Zusammensetzung in einer Form ausgewählt aus der Gruppe bestehend aus Lebensmittelzusätzen, Lebensmittelmaterialien, Lebensmitteln und Getränken, Gesundheitslebensmitteln und -getränken, pharmazeutischen Produkten und Futterstoffen in Mischung mit D-Psicose oder D-Psicose und D-Tagatose als dem aktiven Inhaltsstoff ist.

3. Zusammensetzung für die Verwendung in der Vermeidung des Einsetzens und der therapeutischen Behandlung von gestörten Zuständen in Assoziation mit der Glucokinaseaktivität nach Anspruch 2, wobei die Zusammensetzung in der Form eines pharmazeutischen Produkts ist und D-Psicose oder D-Psicose und D-Tagatose zusammen mit einem oder mehreren pharmazeutisch akzeptablen Trägern enthält.

4. Zusammensetzung für die Verwendung in der Vermeidung des Einsetzens von gestörten Zuständen in Assoziation mit der Glucokinaseaktivität und der therapeutischen Behandlung der gestörten Zustände nach einem der Ansprüche 1 bis 3, wobei die Menge an D-Psicose oder D-Psicose und D-Tagatose als die wirksame Menge davon 0,5 bis 50 g täglich ist.

## Revendications

1. Composition contenant, en tant que principes actifs, du D-psicose ou du D-psicose et du D-tagatose, pour une utilisation dans la prévention de l'apparition et le traitement thérapeutique d'états de troubles en association avec l'activité de glucokinase, choisis parmi une détérioration de la tolérance au glucose, le diabète sucré de type 2, l'hyperlipidémie, le syndrome métabolique, et l'obésité.

2. Composition pour une utilisation dans la prévention de l'apparition et le traitement thérapeutique d'états de troubles en association avec l'activité de glucokinase selon la revendication 1, laquelle composition est sous une forme choisie dans le groupe constitué par les additifs alimentaires, les produits alimentaires, les aliments et les boissons, les aliments et boissons de santé, les produits pharmaceutiques et les aliments pour animaux en mélange avec du D-psicose ou du D-psicose et du D-tagatose en tant que principe actif.

3. Composition pour une utilisation dans la prévention de l'apparition et le traitement thérapeutique d'états de troubles en association avec l'activité de glucokinase selon la revendication 2, laquelle composition est sous la forme d'un produit pharmaceutique et contient du D-psicose ou du D-psicose et du D-tagatose conjointement avec un ou plusieurs véhicules pharmaceutiquement acceptables.

4. Composition pour une utilisation dans la prévention de l'apparition d'états de troubles en association avec l'activité de glucokinase et le traitement thérapeutique des états de troubles selon l'une quelconque des revendications 1 à 3, dans laquelle la quantité de D-psicose ou de D-psicose et de D-tagatose, en tant que quantité efficace de ceux-ci, est de 0,5 à 50 g par jour.
